# EUROPEAN PATENT SPECIFICATION

(11) **EP 3 443 894 B1**
(45) Date of publication and mention of the grant of the patent: **08.12.2021**
(21) Application number: 17782245.9
(22) Date of filing: 29.03.2017
(51) Int. Cl.: A61B 5/02, A61B 5/0245, A61B 5/021, A61B 5/00

(54) **PULSE WAVE DETECTION DEVICE AND BIOLOGICAL INFORMATION MEASURING DEVICE**
PULSWELLENDETEKTOR UND VORRICHTUNG ZUR MESSUNG BIOLOGISCHER INFORMATIONEN
DISPOSITIF DE DÉTECTION D'ONDES PULSATILES ET DISPOSITIF DE MESURE D'INFORMATIONS BIOLOGIQUES

(30) Priority: 15.04.2016 JP 2016082370
(43) Date of publication of application: 20.02.2019
(73) Proprietor: Omron Healthcare Co., Ltd., Muko-shi, Kyoto 617-0002 (JP)
(72) Inventor: FUJITA Reiji, Muko-shi Kyoto 617-0002 (JP); TANAKA Shinya, Muko-shi Kyoto 617-0002 (JP)
(74) Representative: Isarpatent
(86) International application number: PCT/JP2017/012942
(87) International publication number: WO 2017/179427

(56) References cited:
- JP-A- H0 852 118
- JP-A- H0 852 118
- JP-A- 2003 210 424
- JP-A- 2004 305 629
- JP-A- 2008 168 054
- JP-A- 2009 240 511
- JP-A- 2015 503 933

## Description

### Technical Field

The present invention relates to a pulse wave detector and a biometric information measurement device. In addition, an attachment auxiliary member for a pulse wave detector is described.

### Background Art

A biometric information measurement device is known that, in a state where a sensor is directly contacted with a living body portion through which an artery such as the radial artery in the wrist passes, can measure biometric information such as the heart rate, the pulse rate, or the blood pressure by using information detected by the sensor (for example, see Patent Literatures 1 and 2).

JP-A-2008-168054 discloses a biometric information measurement device which is attached to the wrist by using only a housing accommodating a sensor. In the housing of the biometric information measurement device, an opening for avoiding the ulna is disposed in a portion that is to be wound around the back side of the hand in a state where the device is attached to the wrist. This opening enables the attachment state of the device to the wrist, to be stably maintained.

JP-A-S51-041285 discloses a biometric information measurement device in which both end portions of a band portion that is to be wound around the wrist are divided into three parts, and one and other ends of the divided band parts are enabled to be respectively secured.

Document JP H08 52118 A describes a portable sphygmograph comprising a housing and a band configured to attach the housing to a wrist of a subject. An elastic member is provided detachable from the band, wherein in the elastic member serves to press against the subject in order to increase a force that presses a sensor of the sphygmograph against subject.

### Summary of Invention

### Technical Problem

In the biometric information measurement device of JP-A-2008-168054, it is necessary to contrive the material and shape of the housing, and therefore, the production cost of the device is increased. A configuration where the housing is secured to the wrist by using a band is effective for suppressing the production cost. In the wrist, however, there is a portion where the ulna protrudes. When the band is to be fastened, therefore, the band hits the ulna, and hence there arises a possibility that the feeling and easiness of attachment of the device may be impaired, or after attachment, displacement of the position of the device may occur.

When a highly stretchable material is used in the band, the feeling and easiness of attachment of the device can be improved. However, in the case of a band having a high stretchability, after the device is secured to the wrist, there arises a possibility that displacement of the position of the sensor portion may be caused by stretching or contraction of the band portion, and therefore, it is difficult to accurately measure biometric information.

In the biometric information measurement device of JP-A-S51-041285, sensors are secured to the divided parts of the band, respectively, thereby enabling the pressing positions of the sensors to be adjustable. However, the sensors are disposed on the band itself, and therefore, there is a high possibility that the positions of the sensors may be displaced by a motion of the hand. As a result, the device cannot accurately measure biometric information.

The above-described problems are not limited in a biometric information measurement device which detects a pressure pulse wave by using a pressure sensor or the like, and similarly occur also in a biometric information measurement device in which, for example, a volume pulse wave is detected by using a photoelectric sensor.

The present invention has been made in view of the above circumstances. It is an object of the present invention to provide a pulse wave detector in which the feeling and easiness of attachment to the wrist can be improved, and which can accurately detect a pulse wave and a biometric information measurement device including the detector. In addition, an attachment auxiliary member which is to be used in a pulse wave detector is described.

### Means for Solving Problem

The invention provides a pulse wave detector in accordance with claim 1. A pulse wave detector according to the present disclosure is used while being attached to a wrist of a measurement subject and includes: a body portion which includes a detecting section configured to detect a pulse wave from a radial artery of the measurement subject; a strip-like band which is configured to secure the body portion to the wrist; and an attachment auxiliary member which is configured to be attachable to and detachable from the band, wherein in a secured state where the body portion is secured to the wrist with the band to which the attachment auxiliary member is attached, the attachment auxiliary member is contacted with a vicinity of an ulna or radius of the wrist.

The invention further provides a biometric information measurement device in accordance with claim 5. A biometric information measurement device according to the present disclosure includes: the above pulse wave detector; and a biometric information calculating section which is configured to calculate biometric information based on the pulse wave detected by the pulse wave detector.

An attachment auxiliary member according to the present disclosure is for a pulse wave detector including a body portion which includes a detecting section configured to detect a pulse wave from a radial artery of a wrist of a measurement subject; and a strip-like band which is configured to secure the body portion to the wrist, wherein the attachment auxiliary member is configured to be attachable to and detachable from the band, and wherein in a secured state where the body portion is secured to the wrist with the band to which the attachment auxiliary member is attached, the attachment auxiliary member is contacted with a vicinity of an ulna or radius of the wrist.

### Advantageous Effects of Invention

According to the present invention, it is possible to provide a pulse wave detector in which the feeling and easiness of attachment to the wrist can be improved, and which can accurately detect a pulse wave and a biometric information measurement device including the detector. In addition, an attachment auxiliary member which is to be used in a pulse wave detector is described.

### Brief Description of Drawings

Fig. 1 is a perspective view schematically showing the external configuration of a biometric information measurement device 100 of an embodiment of the present invention.
Fig. 2 is a side view of the biometric information measurement device 100 shown in Fig. 1, as seen from the side of the elbow of the left hand of a measurement subject (the central side of the measurement subject).
Fig. 3 is a plan view schematically showing a band 40 to which an attachment auxiliary member 30 is attached.
Figs. 4A and 4B are perspective views schematically showing the external configuration of the attachment auxiliary member 30.
Figs. 5A and 5B are side views of the attachment auxiliary member 30.
Figs. 6A and 6B are perspective views schematically showing the external configuration of an attachment auxiliary member 50 which is a modification of the attachment auxiliary member 30.
Figs. 7A and 7B are perspective views schematically showing the external configuration of an attachment auxiliary member 30A which is a modification of the attachment auxiliary member 30 of the biometric information measurement device 100 shown in Fig. 1.
Fig. 8 is a view showing a modification of the attachment auxiliary member 30 of the biometric information measurement device 100 shown in Fig. 1.
Fig. 9 is a view showing a modification of an attachment position of the attachment auxiliary member 30 of the biometric information measurement device 100 shown in Fig. 1.

### Description of Embodiments

Hereinafter, embodiments of the present invention will be described with reference to the drawings.

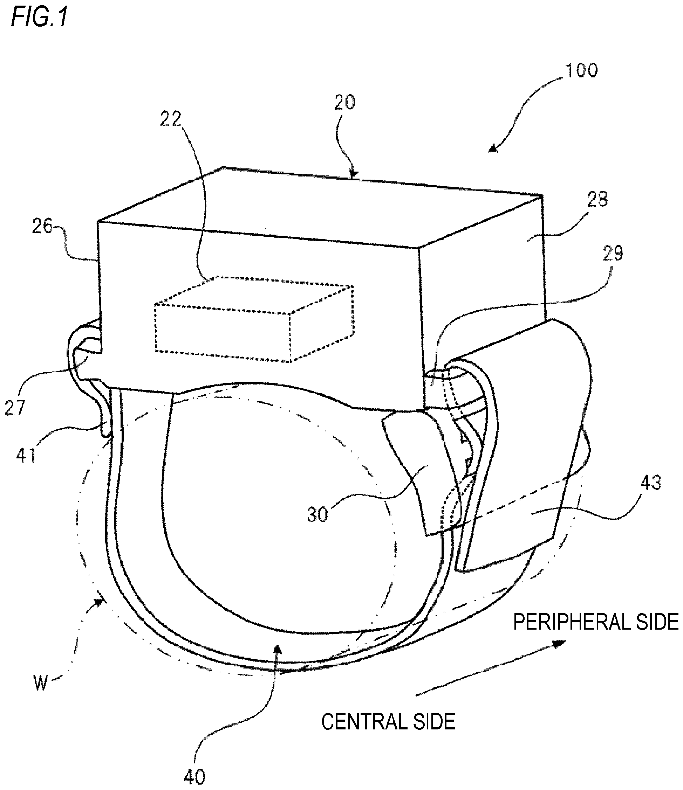

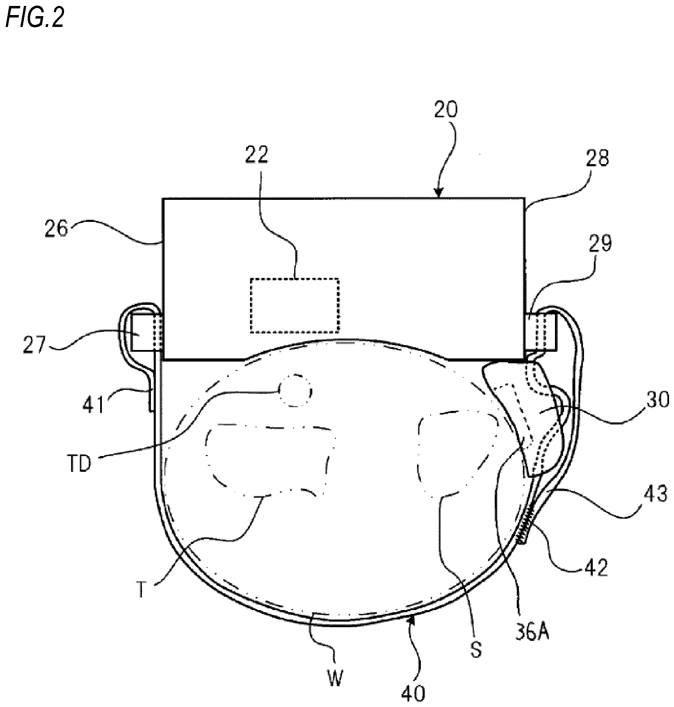

Fig. 1 is a perspective view schematically showing the external configuration of a biometric information measurement device 100 of an embodiment of the present invention. As shown in Fig. 1, the biometric information measurement device 100 is used while being attached to the wrist W of the left hand of a measurement subject. Fig. 2 is a side view of the biometric information measurement device 100 shown in Fig. 1, as seen from the side of the elbow of the left hand of the measurement subject (the central side of the measurement subject). Although the example in which the biometric information measurement device 100 is attached to the wrist of the left hand will be described, the biometric information measurement device 100 can be attached also to the wrist of the right hand.

The biometric information measurement device 100 has: a housing 20 which is formed of a metal or a resin; a strip-like band 40 which is a member for securing the housing 20 to the wrist W; and an attachment auxiliary member 30 for assisting the attachment of the housing 20 to the wrist W.

The housing 20 includes a pulse wave detecting section 22 which can detect a pulse wave (a pressure pulse wave or a volume pulse wave) from the radial artery TD that extends along the radius T in the wrist W of the measurement subject. The housing 20 constitutes the body portion of the biometric information measurement device 100. The body portion of the biometric information measurement device 100 may be configured only by the housing 20, or have a configuration where the housing 20 and a sub-housing incorporating batteries or the like are coupled to each other (a configuration where the portion is formed by a plurality of housings).

The pulse wave detecting section 22 includes at least a sensor which can output a pulse wave as a signal. For example, the pulse wave detecting section 22 has a pressure sensor, and a pressing mechanism which presses it against the skin, and detects a pressure pulse wave by using the pressure sensor. Alternatively, the pulse wave detecting section 22 has a photoelectric sensor, and detects a volume pulse wave from a signal detected by the photoelectric sensor.

In the example of Fig. 1, the housing 20 is a member having an approximately box-like shape, and includes the pulse wave detecting section 22, and a biometric information calculating section (not shown) which calculates biometric information such as the heart rate, the pulse rate, or the blood pressure value based on the pulse wave detected by the pulse wave detecting section 22.

The biometric information calculating section may be disposed in an apparatus other than the biometric information measurement device 100. Namely, the housing 20 (the body portion) of the biometric information measurement device 100 may need to have at least the pulse wave detecting section 22. In this case, the biometric information measurement device 100 functions as the pulse wave detector.

The surface of the housing 20 which, in the case where the biometric information measurement device 100 is attached to the wrist W, is opposed to the wrist W constitutes a detection surface. The detection surface may have an approximately flat shape, or a part or the whole of the surface may be curved so as to extend along the outer shape of the wrist W. The pulse wave detecting section 22 is disposed in a position which is opposed to the radial artery TD in a state where the detection surface is opposed to the wrist W.

A first engaging portion 29 is disposed on a first end portion 28 (in the example of Figs. 1 and 2, an end surface of the housing 20 on the side of the ulna S) which is one of both end portions of the housing 20 on the side of the ulna S in the circumferential direction of the wrist W.

A second engaging portion 27 is disposed on a second end portion 26 (in the example of Figs. 1 and 2, an end surface of the housing 20 on the side of the radius T) which is one of the end portions of the housing 20 on the side of the radius T in the circumferential direction of the wrist W.

The first engaging portion 29 and the second engaging portion 27 are used for causing the band 40 to engage with the housing 20. Each of the first engaging portion 29 and the second engaging portion 27 has a shape that has a hole portion into which the band 40 can be inserted.

The band 40 is wound around the wrist W in a state where a basal end portion 41 is engaged with the second engaging portion 27 of the housing 20 (routed so as to form a state where the wrist W is sandwiched between the band 40 and the housing 20), thereby securing the housing 20 to the wrist W while maintaining the state where the pulse wave detecting section 22 is opposed to the radial artery TD.

For example, the band 40 is a member which is lower in rigidity than the housing 20. As the material of the band 40, for example, cloth, leather, rubber, thin resin, or the like may be used.

The basal end portion 41 which constitutes one end of the band 40 in the longitudinal direction is engaged in a state where the tip end portion is folded back in the second engaging portion 27 which is disposed on the second end portion 26.

A tip end portion 43 which constitutes the other end of the band 40 in the longitudinal direction is engaged in a state where the tip end portion is folded back in the first engaging portion 29 which is disposed on the first end portion 28.

Specifically, the tip end portion 43 of the band 40 is inserted into the hole portion of the first engaging portion 29, in a direction from the side of the detection surface. The tip end portion 43 which has passed through the hole portion is folded back in an approximately U-like shape in a direction to be separated from the housing 20. A hook and loop fastener 42 is formed on the surface of the non-folded back part of the tip end portion 43 and directed in the direction opposite to the housing 20, and the surface of the folded back part of the tip end portion 43 and directed to the housing 20. Because of the hook and loop fastener 42, the folded back part of the tip end portion 43 is detachably engaged with the band 40.

The hook and loop fastener 42 is an engagement member for causing the tip end portion 43 to detachably engage with the band 40. A configuration may be employed where the tip end portion 43 is detachably engaged with the band 40 by using a concave portion and a convex portion which can be fitted thereinto.

The attachment auxiliary member 30 is a member which is configured so as to be attachable to and detachable from the band 40. Specifically, the attachment auxiliary member 30 has a through hole through which the band 40 can be passed. While the band 40 is passed through the through hole, the attachment auxiliary member 30 is attached to the band 40. Preferably, the attachment auxiliary member 30 is configured by a material which allows the band 40 to be easily passed therethrough (for example, wood, metal, or resin).

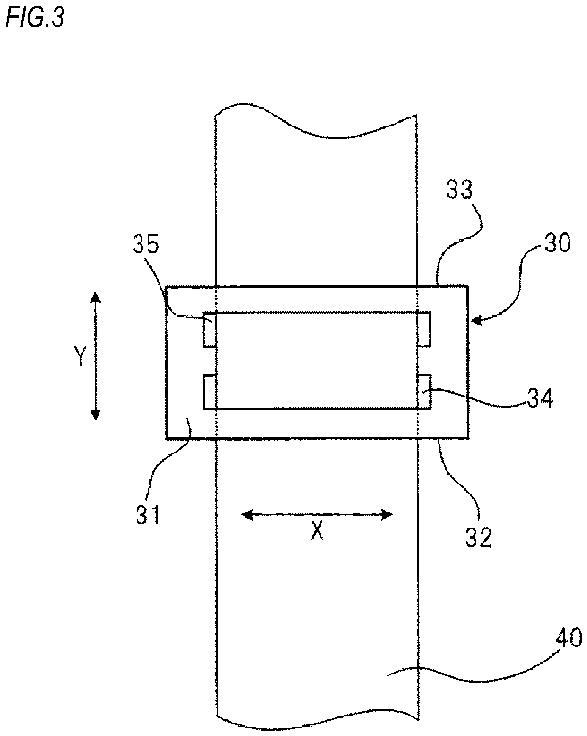

The configuration of the attachment auxiliary member 30 will be described in detail later with reference to Figs. 3 to 5.

Fig. 3 is a plan view schematically showing the band 40 to which the attachment auxiliary member 30 is attached. The direction Y shown in Fig. 3 indicates the longitudinal direction of the band 40. The direction X shown in Fig. 3 indicates the short-side direction perpendicular to the longitudinal direction of the band 40.

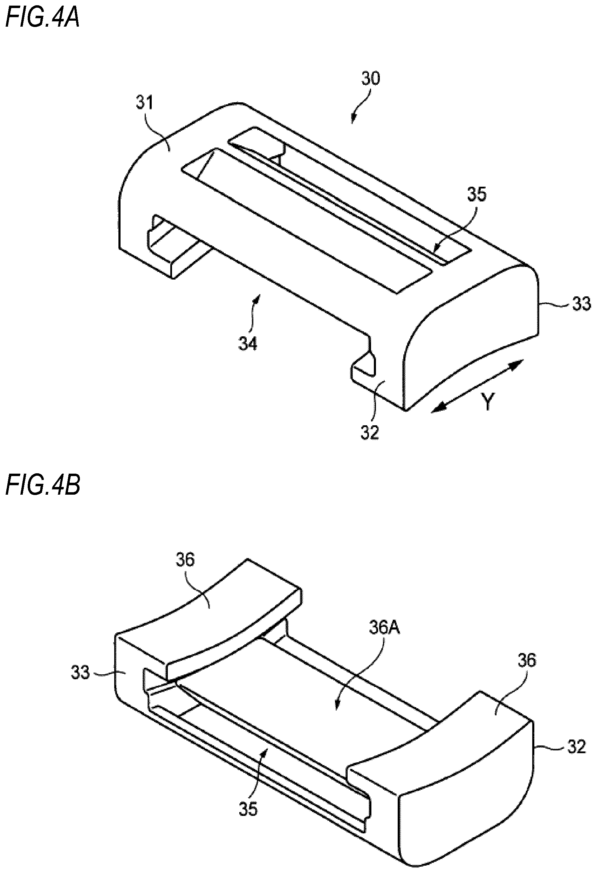

Figs. 4A and 4B are perspective views schematically showing the external configuration of the attachment auxiliary member 30. Fig. 4A is a perspective view of the attachment auxiliary member 30, as seen from the side opposite to that opposed to the wrist W in a state where the housing 20 is secured to the wrist W by the band 40 to which the attachment auxiliary member 30 is attached (hereinafter, referred to as the secured state). Fig. 4B is a perspective view of the attachment auxiliary member 30, as seen from the side opposed to the wrist W in the secured state.

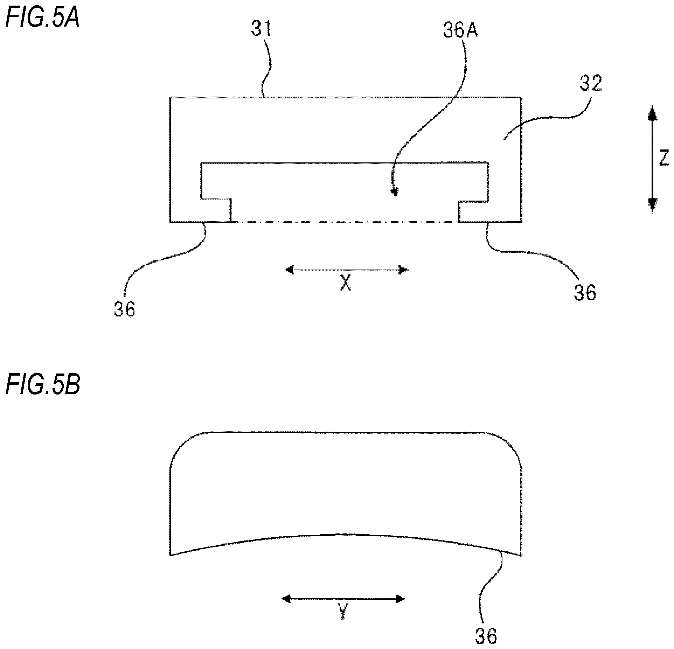

Figs. 5A and 5B are side views of the attachment auxiliary member 30. Fig. 5A is a view of the attachment auxiliary member 30, as seen in the direction Y in Fig. 3.

Fig. 5B is a view of the attachment auxiliary member 30, as seen in the direction X in Fig. 3. In Fig. 5, the direction Z (the direction in which the member is pressed against the skin in the secured state) which is perpendicular to both the directions X and Y shown in Fig. 3.

As shown in Fig. 4, the general shape of the attachment auxiliary member 30 is similar to a shape which is obtained by dividing a column into halves. In the front surface 31 of the attachment auxiliary member 30, a through hole 34 which reaches one side surface 32 of the attachment auxiliary member 30 in the direction Y, and a through hole 35 which reaches the other side surface 33 of the attachment auxiliary member 30 in the direction Y are formed. The band 40 which is inserted into the through hole 34 is passed through the through hole 35, and then pulled out from the side surface 33. Accordingly, the attachment auxiliary member 30 is attached to the band 40 in a state where the member is movable in the direction Y.

The rear surface 36 which is opposite to the front surface 31 of the attachment auxiliary member 30 is a portion which is opposed to the wrist W of the measurement subject in the secured state. A concave portion 36A is formed in the rear surface 36. The concave portion 36A has a size which allows the protrusion of the ulna S in the wrist W of the measurement subject to be received.

In the secured state, the attachment auxiliary member 30 is used while being placed in a position which is contacted with the vicinity of the ulna S in the wrist W. The vicinity of the ulna S in the wrist W means a range where the ulna S can be touched with the finger.

The existence of the concave portion 36A enables the protrusion of the ulna S to be received in the concave portion 36A even in a state where the attachment auxiliary member 30 is contacted with the skin of the vicinity of the ulna S. Therefore, interference between the protrusion of the ulna S and the attachment auxiliary member 30 can be prevented from occurring, and the attachability of the biometric information measurement device 100 can be improved.

As shown in Fig. 5A, the rear surfaces 36 of both end portions of the attachment auxiliary member 30 in the direction X are identical with each other in position in the the direction Z.

As shown in Fig. 5B, the rear surfaces 36 which are in contact with the wrist W in the secured state are curved surfaces that are curved along the circumferential direction of the wrist W. According to the configuration, the contactability between the rear surfaces 36 and the wrist W can be made satisfactory, and the attachability of the biometric information measurement device 100 can be improved.

An example of a method of attaching the thus configured biometric information measurement device 100 to the wrist W will be described.

As shown in Fig. 3, firstly, the measurement subject attaches the attachment auxiliary member 30 to the band 40 such that the rear surfaces 36 of the attachment auxiliary member 30 are directed to the skin. Then, the measurement subject inserts the tip end portion 43 of the band 40 into the hole portion of the first engaging portion 29 of the housing 20 from the side of the detection surface. In this state, the measurement subject finely adjusts the position of the attachment auxiliary member 30 by moving the attachment auxiliary member 30 in the longitudinal direction of the band 40, so that the protrusion of the ulna S enters the concave portion 36A of the attachment auxiliary member 30. After the adjustment, the measurement subject folds back and pulls the tip end portion 43 which has passed through the hole portion of the first engaging portion 29, thereby adjusting the degree of fastening of the band 40. Finally, the measurement subject causes the tip end portion 43 to engage with the band 40 through the hook and loop fastener 42, thereby completing the attachment of the biometric information measurement device 100.

According to the biometric information measurement device 100, as described above, a pressure which is applied from the protrusion of the ulna S to the band 40 can be absorbed by the concave portion 36A of the attachment auxiliary member 30. Therefore, a situation where the ulna S interferes the fastening of the band 40 to worsen the feeling and easiness of attachment of the biometric information measurement device 100 does not occur, and the feeling and easiness of attachment can be improved. Moreover, a further situation where, after attachment of the biometric information measurement device 100, the position of the device is displaced can be prevented from occurring. According to the biometric information measurement device 100, the material of the band 40 is not limited. Therefore, a material having an ideal stretchability for securing of the housing 20 can be employed, displacement of the position of the device can be prevented from occurring, and the accuracy of detection of a pulse wave can be improved.

According to the biometric information measurement device 100, the attachment auxiliary member 30 is placed between the wrist W and the band 40. Therefore, even in the case where a gap is formed between the wrist W and the band 40, the gap is filled by the attachment auxiliary member 30. Accordingly, the biometric information measurement device 100 can be closely contacted with the wrist W, and it is possible to prevent displacement of the position of the device from occurring after attachment of the biometric information measurement device 100. As a result, the accuracy of detection of a pulse wave can be improved.

According to the biometric information measurement device 100, the rear surfaces 36 of the attachment auxiliary member 30 which are in contact with the wrist W in the secured state are curved along the circumferential direction of the wrist W. Therefore, the close contactness between the attachment auxiliary member 30 and the wrist W can be improved, and the feeling of attachment of the biometric information measurement device 100 can be improved.

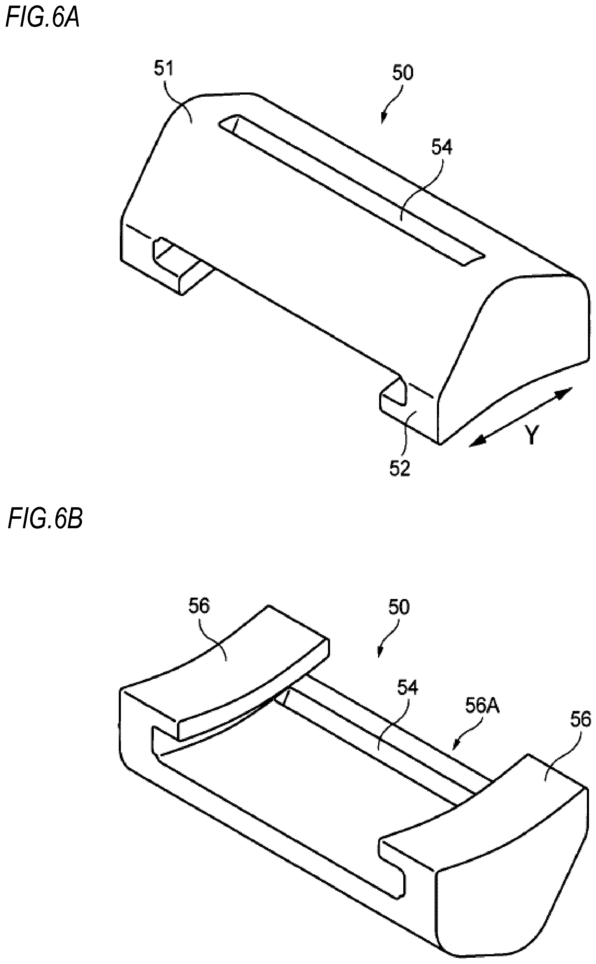

The configuration of the attachment auxiliary member 30 shown in Fig. 3 is an example, and not limited thereto. For example, the attachment auxiliary member 30 may have the configuration such as shown in Figs. 6A and 6B.

Figs. 6A and 6B are perspective views schematically showing the external configuration of an attachment auxiliary member 50 which is a modification of the attachment auxiliary member 30.

The general shape of the attachment auxiliary member 50 has a shape similar to that which is obtained by dividing a column into halves. In the front surface 51 of the attachment auxiliary member 50, a through hole 54 which reaches a side surface 52 of the attachment auxiliary member 50 in the direction Y is formed. The band 40 which is inserted into the through hole 54 from the side of the side surface 52 is pulled out from the side of the front surface 51. Therefore, the attachment auxiliary member 50 is attached to the band 40 in a state where the member is movable in the direction Y.

The rear surface 56 which is opposite to the front surface 51 of the attachment auxiliary member 50 is a portion which is opposed to the wrist W of the subject in the secured state. A concave portion 56A is formed in the rear surface 56. The concave portion 56A has a size which allows the protrusion of the ulna S in the wrist W of the measurement subject to be inserted. The concave portion 56A functions also as a part of the through hole 54.

The above-described effects can be attained also in the case where the attachment auxiliary member 50 shown in Figs. 6A and 6B is used in place of the attachment auxiliary member 30.

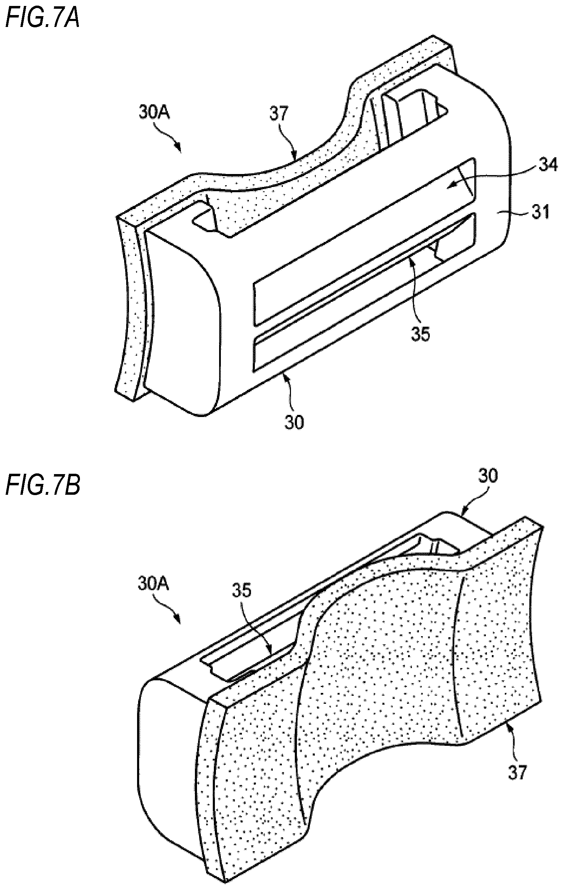

Figs. 7A and 7B are perspective views schematically showing the external configuration of an attachment auxiliary member 30A which is a modification of the attachment auxiliary member 30 of the biometric information measurement device 100 shown in Fig. 1.

The attachment auxiliary member 30A shown in Fig. 7 has a configuration where a planar elastic member 37 is secured to the rear surface 36 of the attachment auxiliary member 30 by an adhesive material or the like.

The elastic member 37 is secured to the rear surface 36 while covering the concave portion 36A of the attachment auxiliary member 30, and in a portion overlapping with the concave portion 36A, has a shape which is recessed toward the concave portion 36A. Because of the recessed shape, a configuration is formed where the insertion of the protrusion of the ulna S into the concave portion 36A is not impeded. In the case where a material having a high stretchability is used as the elastic member 37, the recess is not essential.

The front surface of the elastic member 37 which is on the rear surfaces 36 of the attachment auxiliary member 30 follows the curved shape of the rear surfaces 36 to be formed as a curved surface.

Since the elastic member 37 is a portion which is to be contacted with the skin, the feeling of attachment of the biometric information measurement device 100 is considered, and the raw material which is lower in rigidity than the attachment auxiliary member 30 is selected as the elastic member 37. For example, rubber, sponge, or the like can be used as the elastic member 37.

According to the attachment auxiliary member 30A shown in Fig. 7, the portion which is to be contacted with the skin is the elastic member 37. Therefore, the contact between the attachment auxiliary member 30A and the skin can be satisfactorily performed, and the feeling of attachment of the biometric information measurement device 100 can be improved.

When a material which exerts a large frictional force with respect to the skin is used as the elastic member 37, an anti-slip function can be added to the attachment auxiliary member 30A, and it is possible to prevent displacement of the position of the biometric information measurement device 100 from occurring after attachment of the device.

The attachment auxiliary member 30 which has been described with reference to Figs. 3 to 5 has the configuration where, as shown in Fig. 5A, the rear surfaces 36 of the both end portions in the direction X have the same position in the direction Z. Preferably, the member is configured so that the Z-direction positions of the rear surfaces 36 of the both end portions are different from each other.

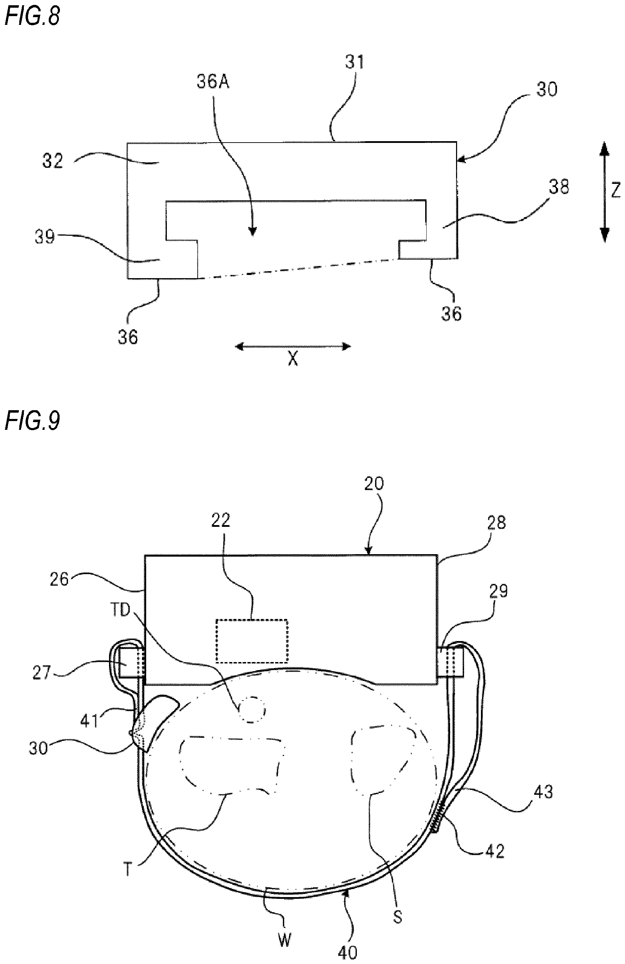

Fig. 8 is a view showing a modification of the attachment auxiliary member 30 of the biometric information measurement device 100 shown in Fig. 1. Fig. 8 is a view showing the attachment auxiliary member 30, as seen in the direction Y shown in Fig. 3.

In the attachment auxiliary member 30 shown in Fig. 8, the rear surface 36 of the end portion (peripheral side end portion) 39 which is placed on the peripheral side in the direction X in the secured state, and the rear surface 36 of the end portion (central side end portion) 38 which is placed on the central side in the direction X in the secured state are different in a position in the direction Z.

When an arbitrary position which is on the side of the front surface 31 with respect to the rear surface 36 of the central side end portion 38 in the direction Z is set as a reference position, specifically, the Z-direction position of the rear surface 36 of the peripheral side end portion 39 is remoter from the reference position than the Z-direction position of the rear surface 36 of the central side end portion 38.

The wrist W gradually becomes thinner as approaching from the central side toward the periphery. Therefore, when the configuration shown in Fig. 8 is employed, it is possible to prevent the attachment auxiliary member 30 from being inclined in the direction Z because of the difference in diameter of the wrist. Consequently, the band 40 can be prevented from being twisted, and the attachability of the biometric information measurement device 100 can be improved.

The attachment auxiliary member 30 of the biometric information measurement device 100 may be enabled to be attached to and detached from the band 40, by using an engagement member such as a hook and loop fastener. In this case, a hook and loop fastener disposed on a surface of the band 40 which is opposed to the wrist, and that disposed on the front surface 31 of the attachment auxiliary member 30 are coupled to each other, whereby the attachment auxiliary member 30 is attached to the band 40.

The attachment auxiliary member 30 may be used while being attached to a position which is contacted with the vicinity of the radius T in the wrist W in the secured state. As shown in Fig. 9, for example, the attachment auxiliary member 30 may be attached to the vicinity of the second engaging portion 27. In the case of the attachment state shown in Fig. 9, the rear surface of the attachment auxiliary member 30 is contacted with the vicinity of the radius T in the wrist W. The vicinity of the radius T in the wrist W means a range of the wrist W where the radius T can be touched with the finger.

In this case, the concave portion 36A has a size which allows the protrusion of the radius T to be received, and therefore, even in the state where the attachment auxiliary member 30 is in contact with the skin in the vicinity of the radius T, the protrusion of the radius T can be received in the concave portion 36A. Consequently, interference between the protrusion of the radius T and the attachment auxiliary member 30 can be prevented from occurring, and the attachability of the biometric information measurement device 100 can be improved.

In the above description, although the attachment auxiliary member 30 has the concave portion 36A in the rear surface, the concave portion 36A is not essential. In the case where the material constituting the rear surface of the attachment auxiliary member 30 is stretchable and has a certain thickness, or in the case where, as shown in Figs. 7A and 7B, an elastic member which is stretchable, and which has a certain thickness is fixed to the rear surface of the attachment auxiliary member 30, for example, the material or the elastic member can absorb a pressure due to the protrusion of the ulna or the radius, even when the concave portion 36A is not disposed. Therefore, interference between a protrusion of a bone and the attachment auxiliary member 30 can be prevented from occurring.

The above-disclosed embodiment should be considered in all respects to be illustrative and not restrictive. The scope of the present invention is represented by the appended claims.

As described above, the following matters are disclosed in the specification.

The disclosed pulse wave detector is used while being attached to a wrist of a measurement subject and includes: a body portion which includes a detecting section configured to detect a pulse wave from a radial artery of the measurement subject; a strip-like band which is configured to secure the body portion to the wrist; and an attachment auxiliary member which is configured to be attachable to and detachable from the band, wherein in a secured state where the body portion is secured to the wrist with the band to which the attachment auxiliary member is attached, the attachment auxiliary member is contacted with a vicinity of an ulna or radius of the wrist.

In the disclosed pulse wave detector, the attachment auxiliary member is formed with a concave portion configured to receive a protrusion of the ulna or radius of the wrist in a portion which is opposed to the wrist in the secured state.

In the disclosed pulse wave detector, a surface of the attachment auxiliary member to be contacted with the wrist is curved along a circumferential direction of the wrist.

In the disclosed pulse wave detector, contact surfaces with the wrist of both end portions of the attachment auxiliary member in a short-side direction of the band in a state of being attached to the band are different in position in a direction perpendicular to both the short-side and longitudinal directions of the band.

In the disclosed pulse wave detector, a surface of the attachment auxiliary member to be contacted with the wrist is covered by an elastic member.

The disclosed biometric information measurement device includes: the above pulse wave detector; and a biometric information calculating section which is configured to calculate biometric information based on the pulse wave detected by the pulse wave detector.

The disclosed attachment auxiliary member for a pulse wave detector including a body portion which includes a detecting section configured to detect a pulse wave from a radial artery of a wrist of a measurement subject; and a strip-like band which is configured to secure the body portion to the wrist, is configured to be attachable to and detachable from the band, and in a secured state where the body portion is secured to the wrist with the band to which the attachment auxiliary member is attached, the attachment auxiliary member is contacted with a vicinity of an ulna or radius of the wrist.

In the disclosed attachment auxiliary member, a concave portion which is configured to receive a protrusion of the ulna or radius of the wrist is formed in a portion which is opposed to the wrist in the secured state.

In the disclosed attachment auxiliary member, a surface of the attachment auxiliary member to be contacted with the wrist is curved along a circumferential direction of the wrist.

In the disclosed attachment auxiliary member, contact surfaces with the wrist of both end portions of the attachment auxiliary member in a short-side direction of the band in a state of being attached to the band are different in position in a direction perpendicular to both the short-side and longitudinal directions of the band.

In the disclosed attachment auxiliary member, a surface which is to be contacted with the wrist is covered by an elastic member.

### Industrial Applicability

The present invention is convenient and effective particularly in application to a blood pressure monitor or the like.

The application is based on Japanese Patent Application No. 2016-082370 filed April 15, 2016.

### Explanation of Reference Numerals

- 100: biometric information measurement device
- 20: housing
- 22: pulse wave detecting section
- 26: second end portion
- 27: second engaging portion
- 28: first end portion
- 29: first engaging portion
- 30, 30A: attachment auxiliary member
- 31: front surface
- 32, 33: side surface
- 34, 35: through hole
- 36: rear surface
- 36A: concave portion
- 37: elastic member
- 38: central side end portion
- 39: peripheral side end portion
- 40: band
- 41: basal end portion
- 42: hook and loop fastener
- 43: tip end portion
- 50: attachment auxiliary member
- 51: front surface
- 52: side surface
- 54: through hole
- 56: rear surface
- 56A: concave portion
- X, Y, Z: direction
- T: radius
- TD: radial artery
- S: ulna
- W: wrist

## Claims

1. A pulse wave detector which is used while being attached to a wrist (W) of a measurement subject, the pulse wave detector comprising:
a body portion (20) which includes a detecting section (22) configured to detect a pulse wave from a radial artery (TD) of the measurement subject;
a strip-like band (40) which is configured to secure the body portion (20) to the wrist (W); and
an attachment auxiliary member (30; 50) which is configured to be attachable to and detachable from the band (40),
wherein the band (40) is contacted with the wrist (W) through the attachment auxiliary member (30; 50) at an attachment position of the attachment auxiliary member (30; 50),
wherein the attachment auxiliary member (30; 50) includes, in a contact surface with the wrist (W), both end portions of the attachment auxiliary member (30; 50) in a short-side direction (X) of the band (40) in a state of being attached to the band (40), and a concave portion (36A; 56A) formed between the both end portions to receive a protrusion of an ulna of the wrist (W),
wherein in a secured state where the body portion (20) is secured to the wrist (W) with the band (40) to which the attachment auxiliary member (30; 50) is attached, the attachment auxiliary member (30; 50) is secured such that the protrusion of the ulna is received in the concave portion (36A; 56A) and the both end portions are contacted with the wrist (W),
wherein the concave portion (36A; 56A) has a size which allows the protrusion of the ulna in the wrist of the measurement subject to be inserted, and
wherein the concave portion (56A) functions as a part of a through hole (54) into which the band (40) is inserted.

2. The pulse wave detector according to claim 1,
wherein a surface (36) of the attachment auxiliary member (30; 50) to be contacted with the wrist (W) is curved along a circumferential direction of the wrist (W).

3. The pulse wave detector according to claim 1 or 2,
wherein contact surfaces with the wrist (W) of the both end portions of the attachment auxiliary member (30; 50) are different in position in a direction (Z) perpendicular to both the short-side (X) and longitudinal directions (Y) of the band (40).

4. The pulse wave detector according to any one of claim 1 to 3,
wherein a surface (36) of the attachment auxiliary member (30; 50) to be contacted with the wrist (W) is covered by an elastic member (37).

5. A biometric information measurement device (100) comprising:
the pulse wave detector according to any one of claims 1 to 4; and
a biometric information calculating section which is configured to calculate biometric information based on the pulse wave detected by the pulse wave detector.

## Patentansprüche

1. Pulswellendetektor, der verwendet wird, während er an einem Handgelenk (W) einer Messperson angebracht ist, wobei der Pulswellendetektor umfasst:
einen Körperabschnitt (20), der eine Detektionssektion (22) umfasst, die dafür eingerichtet ist, eine Pulswelle von einer Speichenarterie (TD) der Messperson zu detektieren;
ein streifenartiges Band (40), das dafür eingerichtet ist, den Körperabschnitt (20) an dem Handgelenk (W) zu befestigen; und
ein Anbringungshilfselement (30; 50), das dafür eingerichtet ist, an dem Band (40) angebracht und von dem Band (40) gelöst werden zu können,
wobei das Band (40) durch das Anbringungshilfselement (30; 50) an einer Anbringungsposition des Anbringungshilfselements (30; 50) mit dem Handgelenk (W) in Kontakt gebracht wird,
wobei das Anbringungshilfselement (30; 50), in einer Kontaktfläche mit dem Handgelenk (W), beide Endabschnitte des Anbringungshilfselements (30; 50) in einer Richtung (X) der kurzen Seite des Bands (40) in einem Zustand, in dem es an dem Band (40) angebracht ist, und einen konkaven Abschnitt (36A; 56A), der zwischen den beiden Endabschnitten ausgebildet ist, um eine Protrusion einer Ulna des Handgelenks (W) aufzunehmen, umfasst,
wobei in einem befestigten Zustand, in dem der Körperabschnitt (20) mit dem Band (40), an dem das Anbringungshilfselement (30; 50) angebracht ist, an dem Handgelenk (W) befestigt ist, das Anbringungshilfselement (30; 50) so befestigt ist, dass die Protrusion der Ulna in dem konkaven Abschnitt (36A; 56A) aufgenommen ist und die beiden Endabschnitte mit dem Handgelenk (W) in Kontakt stehen,
wobei der konkave Abschnitt (36A; 56A) eine Größe aufweist, die es erlaubt, die Protrusion der Ulna in dem Handgelenk der Messperson einzuführen, und
wobei der konkave Abschnitt (56A) als ein Teil eines Durchgangslochs (54) dient, in das das Band (40) eingeführt wird.

2. Pulswellendetektor nach Anspruch 1,
wobei eine Fläche (36) des Anbringungshilfselements (30; 50), die mit dem Handgelenk (W) in Kontakt gebracht werden soll, entlang einer Umfangsrichtung des Handgelenks (W) gekrümmt ist.

3. Pulswellendetektor nach Anspruch 1 oder 2,
wobei Kontaktflächen mit dem Handgelenk (W) der beiden Endabschnitte des Anbringungshilfselements (30; 50) in einer Richtung (Z) senkrecht zu sowohl der Richtung (X) der kurzen Seite als auch der Längsrichtung (Y) des Bandes (40) unterschiedlich positioniert sind.

4. Pulswellendetektor nach einem der Ansprüche 1 bis 3, wobei eine Fläche (36) des Anbringungshilfselements (30; 50), die mit dem Handgelenk (W) in Kontakt gebracht werden soll, durch ein elastisches Element (37) bedeckt ist.

5. Vorrichtung (100) zum Messen biometrischer Informationen, umfassend:
den Pulswellendetektor nach einem der Ansprüche 1 bis 4; und
eine Sektion zum Berechnen biometrischer Informationen, die dafür eingerichtet ist, biometrische Informationen auf der Grundlage der durch den Pulswellendetektor detektierten Pulswelle zu berechnen.

## Revendications

1. Détecteur d'onde puisée qui est utilisé tout en étant attaché à un poignet (W) d'un sujet de mesure, le détecteur d'onde pulsée comprenant :
une portion de corps (20) qui comporte une section de détection (22) configurée pour détecter une onde puisée provenant d'une artère radiale (TD) du sujet de mesure ;
une lanière de type bande (40) qui est configurée pour fixer la portion de corps (20) au poignet (W) ; et
un organe auxiliaire d'attache (30 ; 50) qui est configuré pour pouvoir être attaché à la lanière (40) et détaché de celle-ci,
dans lequel la lanière (40) est mise en contact avec le poignet (W) par le biais de l'organe auxiliaire d'attache (30 ; 50) à une position d'attache de l'organe auxiliaire d'attache (30 ; 50),
dans lequel l'organe auxiliaire d'attache (30 ; 50) comporte, dans une surface de contact avec le poignet (W), deux portions d'extrémité de l'organe auxiliaire d'attache (30 ; 50) dans une direction de côté court (X) de la lanière (40) dans un état d'attache à la lanière (40), et une portion concave (36A ; 56A) formée entre les deux portions d'extrémité pour recevoir une saillie d'un cubitus du poignet (W),
dans lequel dans un état fixé où la portion de corps (20) est fixée au poignet (W) avec la lanière (40) à laquelle l'organe auxiliaire d'attache (30 ; 50) est attaché, l'organe auxiliaire d'attache (30 ; 50) est fixé de sorte que la saillie du cubitus soit reçue dans la portion concave (36A ; 56A) et que les deux portions d'extrémité soient mises en contact avec le poignet (W),
dans lequel la portion concave (36A ; 56A) a une taille qui permet d'insérer la saillie du cubitus dans le poignet du sujet de mesure, et
dans lequel la portion concave (56A) fonctionne comme une partie d'un trou traversant (54) dans lequel la lanière (40) est insérée.

2. Détecteur d'onde pulsée selon la revendication 1,
dans lequel une surface (36) de l'organe auxiliaire d'attache (30 ; 50) à mettre en contact avec le poignet (W) est courbée suivant une direction circonférentielle du poignet (W).

3. Détecteur d'onde pulsée selon la revendication 1 ou 2,
dans lequel des surfaces de contact avec le poignet (W) des deux portions d'extrémité de l'organe auxiliaire d'attache (30 ; 50) sont différentes en position dans une direction (Z) perpendiculaire à la fois à la direction de côté court (X) et à la direction longitudinale (Y) de la lanière (40).

4. Détecteur d'onde puisée selon l'une quelconque des revendications 1 à 3,
dans lequel une surface (36) de l'organe auxiliaire d'attache (30 ; 50) à mettre en contact avec le poignet (W) est couverte par un organe élastique (37).

5. Dispositif de mesure d'informations biométriques (100) comprenant :
le détecteur d'onde puisée selon l'une quelconque des revendications 1 à 4 ; et
une section de calcul d'informations biométriques qui est configurée pour calculer des informations biométriques sur la base de l'onde pulsée détectée par le détecteur d'onde pulsée.
